# EUROPÄISCHE PATENTSCHRIFT

(11) **EP 3 182 894 B1**
(45) Veröffentlichungstag und Bekanntmachung des Hinweises auf die Patenterteilung: **26.12.2018**
(21) Anmeldenummer: 15739215.0
(22) Anmeldetag: 10.07.2015
(51) Int. Cl.: A61B 6/00, G03B 42/04

(54) **EINSCHUBVORRICHTUNG FÜR EINE RÖNTGENKASSETTE**
SLIDE DEVICE FOR AN X-RAY CASSETTE
DISPOSITIF D'INSERTION POUR CASSETTE RADIOGRAPHIQUE

(30) Priorität: 22.08.2014 DE 102014216748
(43) Veröffentlichungstag der Anmeldung: 28.06.2017
(73) Patentinhaber: Gockel-Böhner, Hubert J., 91601 Dombühl (DE)
(72) Erfinder: GOCKEL-BÖHNER, Hubert J., 91601 Dombühl (DE)
(74) Vertreter: Dr. Weitzel & Partner
(86) Internationale Anmeldenummer: PCT/EP2015/065796
(87) Internationale Veröffentlichungsnummer: WO 2016/026613

(56) Entgegenhaltungen:
- DE-U1-202011 002 545
- JP-A- 2006 058 366
- JP-A- 2008 043 479
- US-A- 4 944 053
- Hospital Direct: "X-RAY PLATE INSERTION SYSTEM A SIMPLE BUT EFFECTIVE SYSTEM THAT WILL MAKE A REAL DIFFERENCE Washable Model with new Digital Imager Patient Specific Model", , 23. November 2010 (2010-11-23), XP055216881, Gefunden im Internet: URL:http://www.patient-handling.com/files/ pdf/x_ray.pdf [gefunden am 2015-09-29]

## Beschreibung

Die vorliegende Erfindung betrifft eine Einschubvorrichtung für eine Röntgenkassette zum Einschieben der Röntgenkassette unter eine Person, insbesondere eine liegende Person.

Es gibt Situationen im Krankenhaus oder in der Pflege, in denen eine Person geröntgt werden soll, die in ihrer Mobilität derart eingeschränkt ist, dass das Röntgen mittels eines mobilen Röntgengerätes im Krankenbett, in dem die Person liegt oder sitzt, durchgeführt werden muss. Bei besonders gravierenden Fällen ist es nicht einmal möglich oder zumindest gefährlich, die Person zu bewegen, insbesondere anzuheben.

Zur Aufnahme von Röntgenbildern ist es jedoch erforderlich, eine Röntgenkassette unter dem Bereich der Person zu positionieren, beispielsweise dem Oberkörper, der geröntgt werden soll. Eine solche Röntgenkassette umfasst den eigentlichen Röntgenfilm mit einer Verstärkerfolie, um die für die Belichtung des Films erforderliche Strahlendosis zu reduzieren. Die Verstärkerfolie ist beispielsweise mit einem fluoreszierenden Material beschichtet, das bei Röntgenbestrahlung sichtbares oder ultraviolettes Licht aussendet, das den Film belichtet. In der Regel ist in der Röntgenkassette der eigentliche Röntgenfilm zwischen zwei Verstärkerfolien eingebettet, was auch als Film-Folien-System bezeichnet wird.

Wenn nun die bewegungsunfähige oder -eingeschränkte Person möglichst nicht angehoben werden soll, gestaltet sich bisher das Einschieben der Röntgenkassette unter die Person, das heißt zwischen die Person und das Krankenbett, schwierig, was in der Regel dazu führt, dass die Person doch durch Pflegekräfte beziehungsweise Krankenpfleger angehoben wird, was eigentlich vermieden werden soll.

DE 10 2007 035 625 A1 offenbart eine Röntgenanlage, die Einschubvorrichtungen für eine Röntgenkassette umfasst, die unter einer Person angeordnet sind.

DE 196 27 657 C2 offenbart ein Röntgenaufnahmegerät mit einem Wagen, der über je einen Gelenkarm jeweils einen Röntgenstrahler und einen Flachdetektor dreidimensional einstellbar trägt, wobei der Wagen eine Bildelektronik zur Erzeugung eines Röntgenbildes trägt.

Aus DE 25 03 579 C2 ist eine wiederverwendbare Hülle für eine Röntgenkassette bekannt, wobei die Hülle entlang von drei Kanten miteinander versiegelte zusammenlegbare Folien aufweist. Die US 4,944,053 zeigt eine Einschubvorrichtung mit einer Hülle, wobei die Hülle einen eine Achse umschließenden Schlauch umfasst, der eine innere und eine äußere Lage aufweist.

Der vorliegenden Erfindung liegt die Aufgabe zugrunde, eine Einschubvorrichtung für eine Röntgenkassette zum Einschieben der Röntgenkassette unter eine Person anzugeben, welche die genannte Problematik vermeidet.

Die erfindungsgemäße Aufgabe wird durch eine Einschubvorrichtung mit den Merkmalen von Anspruch 1 gelöst. In den abhängigen Ansprüchen sind vorteilhafte und besonders zweckmäßige Ausgestaltungen der Erfindung sowie ein System zur Positionierung einer Röntgenkassette unter einer insbesondere liegenden Person angegeben.

Eine erfindungsgemäße Einschubvorrichtung für eine Röntgenkassette zum Einschieben der Röntgenkassette, die insbesondere wie eingangs dargestellt ausgeführt ist, unter eine Person weist eine Hülle auf, die einen Aufnahmeraum für die Röntgenkassette umschließt. Die Hülle umfasst einen zu einem Endlosband zusammengefügten Schlauch. Der Schlauch ist dabei derart zu dem Endlosband zusammengefügt, dass er die Form eines glattgedrückten Torus aufweist, sodass vier Lagen eines Materials des Schlauchs übereinander geschichtet sind. In anderen Worten umschließt der Schlauch eine Achse und ist dann in Richtung der Achse zu einem Endlosband zusammengefügt, wobei im zusammengefügten Zustand die Achse zu einem Oval beziehungsweise zu einem Viereck geschlossen ist. Der Schlauch kann beispielsweise auf seiner Innenseite mit einer entlang der Achse verlaufenden Naht, also inneren Quernaht vernäht sein.

Der Aufnahmeraum ist vorteilhaft in einem von dem Endlosband umschlungenen Innenraum positioniert und weist eine Oberseite, eine Unterseite und vier Stirnseiten auf, somit die Form einer Platte oder eines flachen Quaders. Die Oberseite, die Unterseite und zwei zueinander entgegensetzt angeordnete, das heißt voneinander abgewandte Stirnseiten sind durch jeweils eine doppelte Lage des Materials des Schlauches abgedeckt, nämlich ausgehend von dem Aufnahmeraum eine innere Lage gefolgt von einer äußeren Lage.

Die erfindungsgemäße Einschubvorrichtung ermöglicht, dass die Röntgenkassette reibungsarm unter eine Person, insbesondere liegende Person, eingeschoben werden kann, da die Hülle durch Aufeinandergleiten der inneren Lage und der äußeren Lage des Schlauches mitrollt, wenn die Einschubvorrichtung beziehungsweise die Hülle mit einer ihrer beiden Schlauchöffnungen (bezogen auf die Durchgangsöffnungen im Schlauch) in Richtung der Person positioniert wird beziehungsweise ein Stück unter die Person geschoben wird und anschließend die Röntgenkassette auf der entgegengesetzten Seite in die Schlauchöffnung eingeschoben und nachfolgend unter die Person geschoben wird.

Um ein besonders leichtes Gleiten der beiden Lagen des Schlauches aufeinander zu ermöglichen, weist der Schlauch vorteilhaft auf seiner inneren, die Achse umschließenden Oberfläche eine Gleitbeschichtung oder eine Gleitschicht auf.

Zwischen die beiden Lagen des Schlauches kann auf einer Seite, auf zwei entgegengesetzten Seiten, insbesondere oberhalb und unterhalb des Aufnahmeraumes, oder rundum ein Festkörper, insbesondere ein starrer Körper, insbesondere Gleitkörper, eingebracht sein, und/oder der Schlauch kann zwischen den Lagen mit Druckluft befüllt sein oder einem Gel oder Ähnlichem.

Ein solcher Festkörper kann beispielsweise eine Platte sein, insbesondere mit einer Stärke zwischen 0,5 mm und 5 mm. Beispielsweise ist die Platte im Wesentlichen 1 mm dick. Vorteilhaft kann der Festkörper, insbesondere die Platte flexibel sein. Beispielsweise ist eine solche Platte aus PE (Polyethylen) hergestellt.

Gemäß einer Ausführungsform sind zwei solche Platten zwischen die Lagen des Schlauches eingesetzt, jeweils eine Platte auf je einer Seite oberhalb und unterhalb des Aufnahmeraumes.

Gemäß einer Ausführungsform wird die Hülle ausschließlich durch den Schlauch gebildet, das heißt, es existieren nur die genannten vier aufeinanderliegenden Lagen.

Dies schließt nicht aus, dass gemäß einer vorteilhaften Ausführungsform eine Tasche vorgesehen ist, welche den Aufnahmeraum für die Röntgenkassette aufweist und die in ihrer Größe derart an eine von dem Schlauch umschlossene Querschnittsfläche der Schlauchöffnung angepasst ist, dass sie unter Mitnahme von zwei einander zugewandten, die Querschnittsfläche begrenzenden inneren Lagen des Schlauches durch die Schlauchöffnung hindurchschiebbar ist. In diesem Fall wird demnach die Röntgenkassette nicht unummantelt, sozusagen nackt in die Schlauchöffnung der Hülle geschoben, sondern die Röntgenkassette selbst wird zunächst in eine Tasche geschoben, sodass sie auf ihrer Außenseite von der Tasche bedeckt ist und anschließend wird die taschenummantelte Röntgenkassette in die Schlauchöffnung, wie vorstehend beschrieben, eingeschoben, um unter die Person geschoben zu werden. Das Vorsehen einer solchen Tasche bietet den Vorteil, dass die Materialeigenschaften der Tasche auf die Materialeigenschaften der Hülle abgestimmt werden können, damit das Rollen der Hülle optimiert wird, das heißt, dass die innere Lage ideal von der äußeren Oberfläche der Tasche beim Einschieben der Röntgenkassette in die Schlauchöffnung erfasst und mitgezogen wird. Prinzipiell könnte die Röntgenkassette jedoch auch ohne eine solche Tasche in die Schlauchöffnung eingeschoben werden.

Die Tasche kann den Aufnahmeraum vorteilhaft allseitig mit Ausnahme einer Einschuböffnung für die Röntgenkassette umschließen. Die Einschuböffnung kann verschließbar oder nicht verschließbar ausgeführt sein.

Gemäß einer Ausführungsform weist die Tasche wenigstens eine Zugschlaufe auf, die ein Zurückziehen der Tasche erleichtert, wenn die Röntgenkassette nach dem Röntgen wieder aus dem Raum unterhalb der Person herausgezogen werden soll. Vorteilhaft sind mehrere, insbesondere zwei Zugschlaufen vorgesehen, die beispielsweise auf der Seite der Eingriffsöffnung an der Tasche angeschlossen sind.

Die Hülle und/oder die Tasche können vorteilhaft aus einem textilen Material hergestellt sein, das insbesondere elastisch ist. Auch ist es möglich, die Hülle und/oder die Tasche aus einem Einwegmaterial, insbesondere aus Kunststoff herzustellen. Auch Gummi ist möglich. Andere Materialien sind nicht ausgeschlossen.

Ein erfindungsgemäßes System zur Positionierung einer Röntgenkassette unter einer insbesondere liegenden Person umfasst die Röntgenkassette selbst, die in der Regel einen Röntgenfilm eingebettet zwischen zwei Verstärkerfolien aufweist, wie eingangs dargelegt wurde, und ferner die Einschubvorrichtung, wobei die Röntgenkassette in den Aufnahmeraum eingebracht ist. Die Einschubvorrichtung besteht gemäß einer Ausführungsform aus einer Kombination der Hülle und der Tasche.

Sowohl bei dem System als auch bei der Einschubvorrichtung kann die Hülle mit den aufeinander gleitenden Lagen in Richtung der Durchgangsöffnung des Schlauches, das heißt in Richtung der sogenannten Schlauchöffnung, beispielsweise eine Länge von 40 bis 60 cm aufweisen, insbesondere zwischen 45 und 55 cm, beispielsweise von 49 cm. In der Breitenrichtung beträgt die Abmessung vorteilhaft ebenfalls 40 bis 60 cm, insbesondere 45 bis 55 cm, beispielsweise 51 cm.

Die Tasche weist entsprechend vorteilhaft eine Länge auf, bezogen auf die Einschubrichtung in die Schlauchöffnung, die zwischen 30 und 50 cm beträgt, beispielsweise 35 bis 45 cm, insbesondere 40,5 cm. Die sich hieran anschließenden Zugschlaufen können beispielsweise eine Länge von mehr als 10 cm aufweisen, beispielsweise von 19 cm. Die Breite der Tasche beträgt vorteilhaft zwischen 35 und 55 cm, beispielsweise zwischen 40 und 50 cm, insbesondere 47 cm.

Die Erfindung soll nachfolgend anhand eines Ausführungsbeispiels exemplarisch beschrieben werden.

Es zeigen:
- Figur 1: die Hülle einer Einschubvorrichtung vor ihrer Positionierung zum Einschieben einer Röntgenkassette;
- Figur 2: die Hülle aus der Figur 1 mit weiteren Details;
- Figur 3: die Hülle, die zum Einschieben einer Röntgenkassette positioniert ist;
- Figur 4: die beim Einschieben der Röntgenkassette auftretenden Relativbewegungen in der Einschubvorrichtung;
- Figur 5: ein Beispiel für eine Tasche zur Aufnahme der Röntgenkassette.

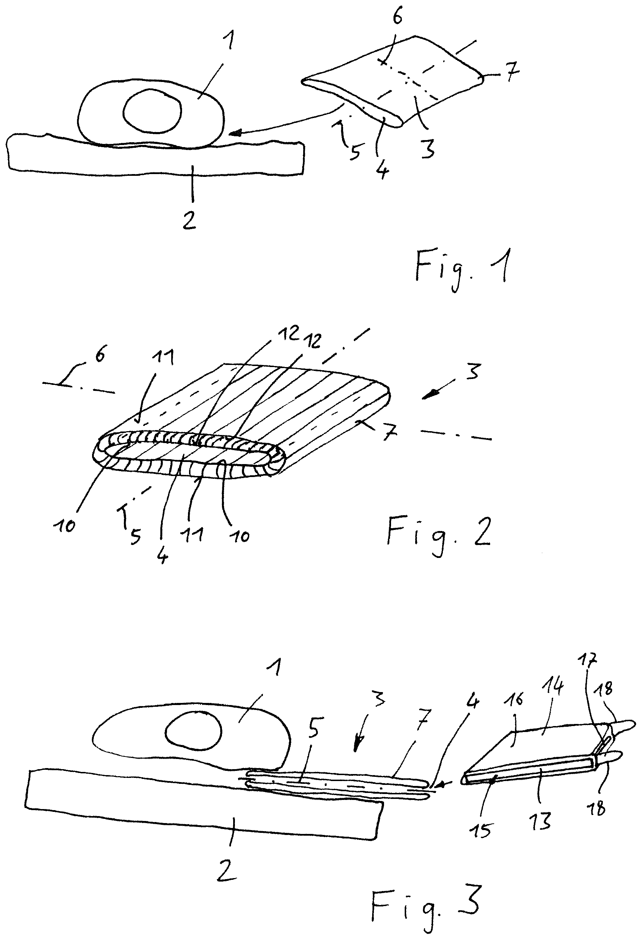

In der Figur 1 ist eine auf einer Unterlage 2, beispielsweise in einem Bett, liegende Person 1 gezeigt. Zwischen die Person 1 und die Unterlage 2 soll nun eine Röntgenkassette mittels einer erfindungsgemäßen Einschubvorrichtung positioniert werden. Hierzu wird zunächst die Hülle 3 mit einer offenen Seite ihrer Schlauchöffnung 4 ein Stück unter die Person 1 geschoben, also in Richtung der hier gezeigten Öffnungsachse 5. Diese Öffnungsachse 5 verläuft senkrecht zur Achse 6, entlang von welcher ein Schlauch 7 der Hülle 3 zu einem Endlosband zusammengefügt ist.

Die Geometrie der Hülle 3, gebildet durch den Schlauch 7, ist nochmals in der Figur 2 dargestellt. So weist der Schlauch 7 vier aufeinanderliegende Lagen auf, nämlich zwei innere Lagen 8 und zwei äußere Lagen 9. Die inneren Lagen 8 bilden ein Endlosband mit einer Durchgangsoberfläche 10, welche die Schlauchöffnung 4 begrenzt. Die äußeren Lagen 9 weisen ebenfalls die Form eines Endlosbandes auf und umschließen die inneren Lagen 8. Die äußeren Lagen 9 bilden die äußere Oberfläche 11 der Hülle 3. Die sogenannte innere, die Achse 6 umschließende Oberfläche des Schlauches 7 ist mit 12 bezeichnet. Diese besteht auf der der Durchgangsoberfläche 10 abgewandten Oberfläche der inneren Lagen 8 und der der äußeren Oberfläche 11 abgewandten Oberfläche der äußeren Lagen 9. Die innere Oberfläche 12 kann beispielsweise mit einer Gleitbeschichtung oder einer Gleitschicht versehen sein, damit die inneren Lagen 8 und die äußeren Lagen 9 besonders leicht aufeinander gleiten, wenn eine Röntgenkassette in Richtung der Öffnungsachse 5 in die Schlauchöffnung 4 eingeschoben wird.

In der Figur 3 ist nun dargestellt, wie die Hülle 3 unter die Person 1 ein Stück weit, beispielsweise zwischen 3 und 10, insbesondere 5 cm eingeschoben wurde und nun eine Röntgenkassette 13 in die Schlauchöffnung 4 auf der der Person 1 abgewandten offenen Seite eingeschoben wird. Im gezeigten Ausführungsbeispiel ist die Röntgenkassette 13 dabei innerhalb einer Tasche 14 positioniert, die einen Aufnahmeraum 15 für die Röntgenkassette 13 umschließt.

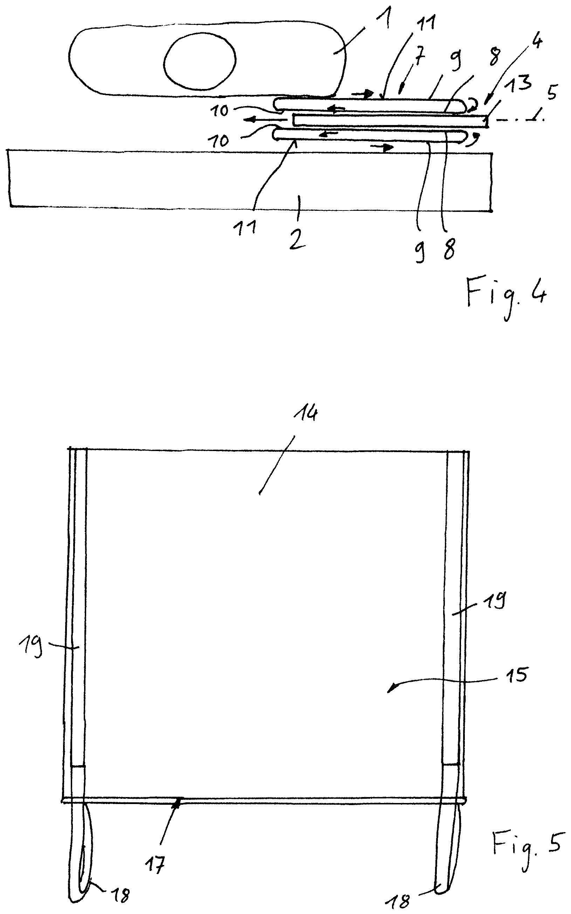

Der Vorteil der Tasche 14 liegt darin, dass diese eine Oberflächenbeschaffenheit auf ihrer äußeren Oberfläche 16 aufweist, die auf die Haft- und Gleiteigenschaften der Durchgangsoberfläche 10 des Schlauches 7 angepasst sind, um die in der Figur 4 näher beschriebenen Relativbewegungen beim Einschieben der Röntgenkassette 13 unter die Person 1 zu erreichen.

Zur vereinfachten Darstellung ist die Röntgenkassette 13 in der Figur 4 ohne Tasche gezeigt, wobei die Tasche ohnehin eine Option darstellt und nicht zwingend notwendig ist.

Wie man anhand der Bewegungspfeile in der Figur 4 erkennen kann, rollen die inneren Lagen 8 beim Einschieben der Röntgenkassette 13 in die Schlauchöffnung 4 in Bewegungsrichtung der Röntgenkassette 13 entlang der Öffnungsachse 5. Die äußeren Lagen 9 hingegen rollen in die entgegengesetzte Richtung. Somit führt der Schlauch 7 in Richtung der Öffnungsachse 5 beziehungsweise in Bewegungsrichtung der Röntgenkassette 13 eine raupenartige Abrollbewegung durch, vergleichbar mit der Bewegung von Ketten einer Raupenkette.

Wie rechts aus der Figur 4 ersichtlich ist, wird durch die raupenartige Bewegung des Schlauches 7 eine Relativbewegung beziehungsweise eine Gleitbewegung zwischen der äußeren Oberfläche 11 des Schlauches 7 und einerseits der Person 1 und andererseits der Unterlage 2 eliminiert oder zumindest minimiert. Zugleich wird das Einschieben der Röntgenkassette 13 durch Reduzierung der Relativbewegung zwischen der Röntgenkassette 13 und der Durchgangsoberfläche 10 erleichtert. Auch hier kann günstigenfalls jegliches Gleiten eliminiert werden.

In der Figur 5 ist nochmals ein Ausführungsbeispiel einer Tasche 14 gezeigt. Diese weist eine Einschuböffnung 17 auf, über welche eine hier nicht dargestellte Röntgenkassette 13 in den dafür vorgesehenen Aufnahmeraum 15 innerhalb der Tasche 14 eingeführt werden kann. Bis auf die Einschuböffnung 17 umschließt die Tasche 14 den Aufnahmeraum 15 vollständig.

Im gezeigten Ausführungsbeispiel weist die Tasche 14, wie auch schon in der Figur 3 angedeutet ist, zwei Zugschlaufen 18 auf, die ein Herausziehen der Tasche 14 mit der Röntgenkassette 13 unter der Person 1 hervor nach dem Röntgen erleichtern, da die Zugschlaufen günstigenfalls auch im vollständig eingeschobenen Zustand der Röntgenkassette 13 unter die Person 1 aus der Schlauchöffnung 4 noch herausragen, zumindest nahe des der Person 1 abgewandten Endes der Schlauchöffnung 4 positioniert und damit leicht greifbar sind. Die Zugschlaufen 18 sind dementsprechend in ihrer Länge dimensioniert.

Günstig ist, wenn die Zugschlaufen 18, beispielsweise zwei Zugschlaufen 18, wie in der Figur 5 gezeigt ist, auf der Seite der Einschuböffnung 17 über den äußeren Umfang der Tasche 14 hervorstehen, beziehungsweise dort an der Tasche 14 angeschlossen sind. Der überstehende Bereich der Zugschlaufen 18 beträgt beispielsweise 10 bis 25 cm, insbesondere 19 bis 20 cm.

Im gezeigten Ausführungsbeispiel in der Figur 5 sind die Zugschlaufen 18 an entlang der Seiten der Tasche 14 verlaufenden aufgenähten Bändern 19 befestigt, um die Haltbarkeit des Anschlusses zu erhöhen.

## Patentansprüche

1. Einschubvorrichtung für eine Röntgenkassette (13) zum Einschieben der Röntgenkassette (13) unter eine Person (1);
mit einer Hülle (3), die einen Aufnahmeraum (15) für die Röntgenkassette (13) umschließt, wobei
die Hülle (3) einen eine Achse (6) umschließenden Schlauch (7) umfasst, der in Richtung der Achse (6) zu einem Endlosband zusammengefügt ist; und
der Schlauch (7) zwei eine Schlauchöffnung (4) zum Einschieben der Röntgenkassette (13) begrenzende innere Lagen (8) und zwei die inneren Lagen (8) umschließende äußere Lagen (9) aufweist,
**dadurch gekennzeichnet, dass**
die inneren Lagen (8) und die äußeren Lagen (9) bei einem Einschieben der Röntgenkassette (13) in die Schlauchöffnung (4) derart aufeinander gleitbar sind, dass sich die inneren Lagen (8) in eine entgegengesetze Richtung wie die äußeren Lagen (9) senkrecht zur Achse (6) bewegen.

2. Einschubvorrichtung gemäß Anspruch 1, **dadurch gekennzeichnet, dass** der Aufnahmeraum (15) in einem von dem Endlosband umschlungenen Innenraum positioniert ist, eine Oberseite, eine Unterseite und vier Stirnseiten aufweist, von denen die Oberseite, die Unterseite und zwei zueinander entgegengesetzt angeordnete Stirnseiten jeweils durch eine doppelte Lage eines Materials, nämlich die innere Lage (8) und die äußere Lage (9) des Schlauches (7) abgedeckt sind.

3. Einschubvorrichtung gemäß einem der Ansprüche 1 oder 2, **dadurch gekennzeichnet, dass** der Schlauch (7) auf seiner inneren, die Achse umschließenden Oberfläche (12) eine Gleitbeschichtung oder Gleitschicht aufweist.

4. Einschubvorrichtung gemäß einem der Ansprüche 1 bis 3, **dadurch gekennzeichnet, dass** die Hülle (3) ausschließlich durch den Schlauch (7) gebildet wird.

5. Einschubvorrichtung gemäß einem der Ansprüche 1 bis 4, **dadurch gekennzeichnet, dass** ferner eine Tasche (14) vorgesehen ist, welche den Aufnahmeraum (15) für die Röntgenkassette (13) aufweist und die in ihrer Größe derart an eine von dem Schlauch (7) umschlossene Querschnittsfläche der Schlauchöffnung (4) angepasst ist, dass sie unter Mitnahme von zwei einander zugewandten, die Schlauchöffnung (4) begrenzenden inneren Lagen (8) des Schlauches (7) durch die Schlauchöffnung (4) hindurchschiebbar ist.

6. Einschubvorrichtung gemäß Anspruch 5, **dadurch gekennzeichnet, dass** die Tasche (14) den Aufnahmeraum (15) mit Ausnahme einer Einschuböffnung (17) allseitig umschließt.

7. Einschubvorrichtung gemäß einem der Ansprüche 5 oder 6, **dadurch gekennzeichnet, dass** die Tasche (14) wenigstens eine Zugschlaufe (18) oder mehrere, insbesondere zwei Zugschlaufen (18) aufweist, die insbesondere auf der Seite der Einschuböffnung (17) vorgesehen sind.

8. Einschubvorrichtung gemäß einem der Ansprüche 1 bis 7, **dadurch gekennzeichnet, dass** die Hülle (3) und/oder die Tasche (14) aus einem textilen Material hergestellt sind, das insbesondere elastisch ist.

9. Einschubvorrichtung gemäß einem der Ansprüche 1 bis 8, **dadurch gekennzeichnet, dass** der Schlauch (7) mit einem Medium oder Druckmedium, wie Luft oder Gel, und/oder mit einem Körper, insbesondere Festkörper gefüllt ist.

10. Einschubvorrichtung gemäß einem der Ansprüche 1 bis 9, **dadurch gekennzeichnet, dass** ein oder zwei starre Körper zwischen die innere Lage (8) und die äußere Lage (9) eingebracht sind.

11. Einschubvorrichtung gemäß einem der Ansprüche 1 bis 10, **dadurch gekennzeichnet, dass** der Schlauch (7) auf seiner Innenseite mit einer entlang der Achse (6) verlaufenden inneren Quernaht vernäht ist.

12. System zur Positionierung einer Röntgenkassette (13) unter einer insbesondere liegenden Person (1), umfassend eine Röntgenkassette (13) und eine Einschubvorrichtung gemäß einem der Ansprüche 1 bis 11, wobei die Röntgenkassette (13) in den Aufnahmeraum (15) eingebracht ist.

## Claims

1. Slide device for an X-ray cassette (13), for sliding the X-ray cassette (13) under a person (1);
having an envelope (3) which encloses a receiving space (15) for the X-ray cassette (13), wherein
the envelope (3) comprises a tube (7) which encloses an axis (6) and which is joined together in the direction of the axis (6) to form a continuous band; and the tube (7) has two inner layers (8) which bound a tube opening (4) for the sliding-in of the X-ray cassette (13), and two outer layers (9) which enclose the inner layers (8),
**characterized in that**,
when the X-ray cassette (13) is being slid into the tube opening (4), the inner layers (8) and the outer layers (9) can slide on one another in such a way that the inner layers (8) move in an opposite direction to the outer layers (9) perpendicular to the axis (6).

2. Slide device according to claim 1, **characterized in that** the receiving space (15) is positioned in an interior, around which the continuous band is wrapped, and has a top side, a bottom side and four end sides, of which the top side, the bottom side and two mutually opposite end sides are each covered by a double layer of a material, namely the inner layer (8) and the outer layer (9) of the tube (7).

3. Slide device according to one of claims 1 or 2, **characterized in that** the tube (7) has a sliding coating or sliding layer on its inner surface (12) which encloses the axis.

4. Slide device according to one of claims 1 to 3, **characterized in that** the envelope (3) is formed exclusively by the tube (7).

5. Slide device according to one of claims 1 to 4, **characterized in that** a pocket (14) is also provided, which has the receiving space (15) for the X-ray cassette (13) and which is adapted in terms of its size to a cross-sectional area of the tube opening (4) that is enclosed by the tube (7), such that it can be pushed through the tube opening (4), thereby entraining two mutually facing inner layers (8) of the tube (7) that bound the tube opening (4).

6. Slide device according to claim 5, **characterized in that** the pocket (14) encloses the receiving space (15) on all sides with the exception of an insertion opening (17).

7. Slide device according to one of claims 5 or 6, **characterized in that** the pocket (14) has at least one pull loop (18) or a plurality of, in particular two, pull loops (18), which are provided in particular on the side of the insertion opening (17).

8. Slide device according to one of claims 1 to 7, **characterized in that** the envelope (3) and/or the pocket (14) are made of a textile material, which in particular is elastic.

9. Slide device according to one of claims 1 to 8, **characterized in that** the tube (7) is filled with a medium or pressure medium, such as air or gel, and/or with a body, in particular solid bodies.

10. Slide device according to one of claims 1 to 9, **characterized in that** one or two rigid bodies are introduced between the inner layer (8) and the outer layer (9).

11. Slide device according to one of claims 1 to 10, **characterized in that** the tube (7) is sewn on its inner side with an inner transverse seam that runs along the axis (6).

12. System for positioning an X-ray cassette (13) under a person (1), in particular a person lying down, comprising an X-ray cassette (13) and a slide device according to one of claims 1 to 11, wherein the X-ray cassette (13) is introduced into the receiving space (15).

## Revendications

1. Dispositif d'introduction pour une cassette radiographique (13), destiné à introduire la cassette radiographique (13) sous une personne (1),
avec une enveloppe (3) qui renferme un espace de logement (15) pour la cassette radiographique (13),
l'enveloppe (3) comprenant une gaine (7) qui entoure un axe (6) et qui est assemblée dans la direction de l'axe (6) pour former une bande sans fin, et
la gaine (7) comportant deux couches intérieures (8) qui délimitent une ouverture de gaine (4) pour l'introduction de la cassette radiographique (13) et deux couches extérieures (9) entourant les couches intérieures (8), **caractérisé en ce que**
les couches intérieures (8) et les couches extérieures (9) peuvent glisser les unes sur les autres lorsque la cassette radiographique (13) est introduite dans l'ouverture de gaine (4), de telle sorte que les couches intérieures (8) se déplacent en sens inverse des couches extérieures (9) perpendiculairement à l'axe (6).

2. Dispositif d'introduction selon la revendication 1, **caractérisé en ce que** l'espace de logement (15) est positionné dans un espace intérieur entouré par la bande sans fin, présente une face supérieure, une face inférieure et quatre faces frontales, la face supérieure, la face inférieure et deux faces frontales disposées à l'opposé l'une de l'autre étant chacune recouverte par une double couche d'un matériau, à savoir par la couche intérieure (8) et la couche extérieure (9) de la gaine (7).

3. Dispositif d'introduction selon l'une des revendications 1 ou 2, **caractérisé en ce que** la gaine (7) présente un revêtement ou une couche facilitant le glissement sur sa surface (12) entourant l'axe.

4. Dispositif d'introduction selon l'une des revendication 1 à 3, **caractérisé en ce que** la l'enveloppe (3) est formée uniquement par la gaine (7).

5. Dispositif d'introduction selon l'une des revendications 1 à 4, **caractérisé en ce qu'**est en outre prévue une poche (14) qui comporte l'espace de logement (15) pour la cassette radiographique (13) et dont la taille est adaptée à une aire de section de l'ouverture de gaine (4) entourée par la gaine (7) de telle manière qu'elle puisse être passée à travers l'ouverture de gaine (4) en entraînant deux couches intérieures (8) de la gaine (7) tournées l'une vers l'autre et délimitant l'ouverture de gaine (4).

6. Dispositif d'introduction selon la revendication 5, **caractérisé en ce que** la poche (14) entoure l'espace de logement (15) de tous côtés, à l'exception d'une ouverture d'introduction (17).

7. Dispositif d'introduction selon l'une des revendications 5 ou 6, **caractérisé en ce que** la poche (14) présente au moins une boucle de traction (18) ou plusieurs boucles de traction (18), en particulier deux, qui sont prévues en particulier du côté de l'ouverture d'introduction (17).

8. Dispositif d'introduction selon l'une des revendications 1 à 7, **caractérisé en ce que** l'enveloppe (3) et/ou la poche (14) sont faites d'une matière textile, qui est en particulier élastique.

9. Dispositif d'introduction selon l'une des revendications 1 à 8, **caractérisé en ce que** la gaine (7) est remplie d'un fluide ou d'un fluide sous pression, tel que de l'air ou du gel, et/ou d'un corps, en particulier d'un corps solide.

10. Dispositif d'introduction selon l'une des revendications 1 à 9, **caractérisé en ce qu'**un ou deux corps rigides sont insérés entre la couche intérieure (8) et la couche extérieure (9).

11. Dispositif d'introduction selon l'une des revendications 1 à 10, **caractérisé en ce que** la gaine (7) est cousue sur sa face intérieure à l'aide d'une soudure transversale intérieure courant le long de l'axe (6).

12. Système de positionnement d'une cassette radiographique (13) sous une personne (1) qui est en particulier couchée, comprenant une cassette radiographique (13) et un dispositif d'introduction selon l'une des revendications 1 à 11, dans lequel la cassette radiographique (13) est introduite dans l'espace de logement (15).
